# EUROPÄISCHE PATENTSCHRIFT

(11) **EP 1 854 431 B1**
(45) Veröffentlichungstag und Bekanntmachung des Hinweises auf die Patenterteilung: **30.12.2009**
(21) Anmeldenummer: 06009804.3
(22) Anmeldetag: 12.05.2006
(51) Int. Cl.: A61F 2/34, A61F 2/40

(54) **Gelenksendoprothese**
Joint endoprosthesis
Prothèse articulaire

(43) Veröffentlichungstag der Anmeldung: 14.11.2007
(73) Patentinhaber: Synthes GmbH, 4436 Oberdorf (CH)
(72) Erfinder: Kropf, Philipp, 6330 Cham (CH)
(74) Vertreter: Lusuardi, Werther

(56) Entgegenhaltungen:
- EP-A- 0 664 108
- EP-A- 1 472 999
- EP-A- 1 639 967
- GB-A- 2 405 346
- US-A1- 2003 055 507

## Beschreibung

Die Erfindung betrifft eine Prothese, insbesondere ein Schulterprothese, mit einem Pfannenteil zur Aufnahme eines Gelenkkopfs, einem Verankerungsteil zum Verankern der Prothese an einem Knochen und einer Verbindungseinrichtung zum festen Verbinden des Pfannenteils mit dem Verankerungsteil. Ferner wird ein Verfahren zum Zusammenbau einer Prothese/offenbart.

Prothesen dieser Art werden üblicherweise bei. Kugelgelenken, insbesondere bei Schulter- und Hüftgelenken, eingesetzt. Beim Schultergelenk bildet eine derartige Prothese eine Glenoid-Komponente zur Aufnahme des Kopfes eines Oberarmknochens (so genannter Humerus).

Aus der Patentschrift US 5,702,447 A ist eine Glenoid-Prothese bekannt, die einen flach ausgeführten Metallsockel zur Verschraubung in einem Schulterblattknochen aufweist. Der Metallsockel ist an seiner Innenseite mit einer Rippe versehen. Ein muldenförmiges Kunststoffpfannenteil kann in Schräglage teilweise unter die Rippe geführt und schliesslich durch Hinabdrucken mit dem Metallsockel fest verbunden werden. Die exakte Positionierung des Kunststoffpfannenteils in dem Metallsockel erfordert eine hohe Präzision. Eine sich zwischen dem Kunststoffpfannenteil und dem Metallsockel befindliche Substanz, wie beispielsweise Blut oder Gewebeteile, kann ein Zustandekommen der Verbindung verhindern. Durch eine einfache Kippbewegung kann das Kunststoffpfannenteil aus dem Metallsockel gelöst werden und die Prothese ist nicht mehr funktionsfähig. Die zwei Bestandteile der Prothese, Kunststoffpfannenteil und Metallsockel, sind dann vollständig voneinander gelöst und können nicht durch einen einfachen Eingriff wieder fest miteinander verbunden werden.

Aus der Patentschrift US 2003/0055507 ist ebenfalls eine modulare Glenoid-Prothese bekannt.

Aus der Patentanmeldung US 2004/0064189 A1 ist eine Glenoid-Komponente bekannt mit einem Verankerungsteil, das über eine Schraube in einem Knochen verankert wird, und einem Pfannenteil, das über eine an dessen Boden befindliche Einschnappeinrichtung mit dem Verankerungsteil verbunden wird. Beim Zusammenfügen des Pfannenteils mit dem Verankerungsteil kann sich das Pfannenteil jedoch im Verankerungsteil verkanten bzw. verkippen, so dass eine feste Verbindung der beiden Teile erschwert wird und eine hohe Präzision erforderlich ist. Weiter verhindern zwischen Pfannenteil und Verankerungsteil gegebenenfalls befindliche Gewebeteile und Blut eine feste Verbindung.

Vor diesem Hintergrund besteht die Aufgabe der vorliegenden Erfindung darin, eine Prothese zu schaffen, mit der die vorgenannten Probleme überwunden werden können.

Diese Aufgabe wird von der eingangs genannten Prothese dadurch gelöst, dass dem Pfannenteil mehrere, insbesondere zwei Stifte zugeordnet sind und das Verankerungsteil mehrere, insbesondere zwei Führungen aufweist, wobei die Führungen derart ausgestaltet sind, dass die Stifte im Wesentlichen kippsicher in den Führungen gehalten werden können bzw. haltbar sind. Durch eine derartige Ausgestaltung der Führungen können die Stifte kontrolliert in die Führungen eingeführt und in bzw. von diesen geführt und gehalten werden. Hierdurch wird eine haltbare Verbindung zwischen Pfannenteil und Verankerungsteil sichergestellt. Sollte sich die Verbindungseinrichtung öffnen und als Folge davon das Pfannenteil von dem Verankerungsteil getrennt werden, so erfolgt dennoch kein Auseinanderfallen der Prothese, da die dem Pfannenteil zugeordneten Stifte in den Führungen des Verankerungsteils verbleiben.

Die Anzahl der Führungen entspricht vorzugsweise der Anzahl der Stifte. Die Stifte und die Führungen sind vorzugsweise parallel zueinander ausgerichtet. Durch das Vorsehen mehrerer Stifte und Führungen kann ein Verdrehen des Pfannenteils gegenüber dem Verankerungsteil verhindert werden. Die Rotationsstabilität der Prothese wird erhöht.

In der Ausgangsöffnung kann ein Befestigungsmittel, insbesondere eine Schraube, zur Befestigung des Verankerungsteils in einem Knochen vorgesehen sein. Zusätzlich zu einer Befestigung, bei der das Verankerungsteil beispielsweise mittels eines Hammers in einen Knochen hineingeschlagen wird, kann durch das Befestigungsmittel ein besserer Halt erzielt werden.

Der Ausdruck "im Wesentlichen kippsicher" umfasst vorliegend auch Situationen, in denen die Stifte in den Führungen um bis zu 10 Grad, vorzugsweise höchstens um bis zu 1 Grad, zur Achse der Führungen gekippt sind.

Bei einem bevorzugten Ausführungsbeispiel der Erfindung sind die Führungen und die Stifte im Wesentlichen zylindrisch ausgebildet, wobei der Innendurchmesser der Führungen im Wesentlichen dem Aussendurchmesser der Stifte entspricht, d.h. beim Einführen der Stifte in die Führungen gleiten die Aussenseiten der Stifte an den Innenseiten der Führungen entlang. Auf diese Weise können die Stifte besonders kippsicher durch die Führungen geführt und gehalten werden. Ferner kann verhindert werden, dass sich Substanzen wie beispielsweise Blut an den Stiften bzw. an der Führungen absetzen.

Der Ausdruck "im Wesentlichen zylindrisch" umfasst vorliegend Ausgestaltungen, die einen Konus von bis zu 10 Grad, vorzugsweise höchstens bis zu 1 Grad, aufweisen. Feine Vertiefungen oder Nuten in der Zylinderoberfläche sind möglich.

Bei einem weiteren Ausführungsbeispiel der Erfindung ist die Verbindungseinrichtung zum festen Verbinden des Pfannenteils mit dem Verankerungsteil als Einschnappeinrichtung ausgeführt. Unter einer festen Verbindung wird vorliegend eine Verbindung verstanden, bei der eine Kraft von mindestens 20 kp (196,13 Newton) aufgebracht werden muss, um die Verbindung zu lösen. Vorzugsweise sind ein erster Teil der Verbindungseinrichtung an dem Stift in einem Abstand zu dem Pfannenteil und ein zweiter Teil der Verbindungseinrichtung in der Führung in einem Abstand zu einer Einfuhrseite der Führung angeordnet. In die Einfuhrseite der Führung kann der Stift eingeführt werden. Durch die Beabstandung des ersten und des zweiten Teils der Verbindungseinrichtung wird sichergestellt, dass der Stift kippsicher durch die Führung geführt wird, bevor die feste Verbindung zwischen dem Pfannenteil und dem Verankerungsteil durch die Verbindungseinrichtung hergestellt wird. Durch die Beabstandung wird ferner sichergestellt, dass bei einem Öffnen der Verbindungseinrichtung der Stift nicht einfach aus der Führung herausgelöst, sondern in dieser gehalten wird. Eine erneute Verbindung von Pfannenteil und Verankerungsteil ist durch einfaches Drücken des Verankerungsteils erreichbar, so dass der Stift so weit in die Führung hineingedrückt wird, dass der erste Teil mit dem zweiten Teil der Verbindungseinrichtung zusammenwirkt und auf diese Weise eine feste Verbindung von Pfannenteil und Verankerungsteil zustandekommt.

Gemäss einem weiteren bevorzugten Ausführungsbeispiel beträgt der Abstand von dem Pfannenteil der Verbindungseinrichtung, insbesondere zu dessen Pfannenteil abgewandtem Ende, zwischen 3 Millimetern und 12 Millimetern, insbesondere 4 Millimeter. Mit diesen Abstandswerten kann eine besonders sichere Führung und ein besonders sicherer Halt des Stifts durch die Führung selbst bei einem Sichöffnen der Verbindungseinrichtung erzielt werden, ohne dass die Führung derart lang wird, dass zur Verankerung der Prothese in einem Knochen tiefe Bohrungen oder Einschnitte erforderlich würden. Ferner braucht nur eine geringe Kraft aufgebracht zu werden, um das Pfannenteil mit dem Verankerungsteil fest zu verbinden, wobei die erforderliche Kraft insbesondere zwischen 15 kp und 22 kp (147,10 Newton bis 215,75 Newton) liegt. Die Kraft zum Lösen der festen Verbindung zwischen Pfannenteil und Verankerungsteil beträgt mindestens 20 kp (196,13 Newton). Die Vorgaben bezüglich der Auspresskraft sind somit erfüllt.

Gemäss einem weiteren Ausbildungsbeispiel der Erfindung ist der erste Teil der Verbindungseinrichtung, welcher an dem Stift vorgesehen ist, als Öffnung, vorzugsweise als im Wesentlichen ringförmige Nut, ausgebildet. Der zweite Teil der Verbindungseinrichtung ist bevorzugterweise als Verdickung, insbesondere als im Wesentlichen ringförmiger Wulst, ausgebildet. Auf diese Weise lässt sich eine feste Verbindung zwischen dem Pfannenteil und dem Verankerungsteil über den Schaft und die Führung erzielen, indem die Verdickung bzw. der Wulst in der Öffnung bzw. der Nut, einrastet bzw. einschnappt. Die Öffnung ist vorzugsweise an ihren Rändern mit einer Fase versehen, so dass es bei der Einfuhr des Stifts in die Führung zu keiner bzw. nur zu einer geringen Abscherung des Stifts bedingt durch die Verdickung bzw. den zweiten Teil der Verbindungseinrichtung kommen kann.

Gemäss einem weiteren bevorzugten Ausführungsbeispiel der Erfindung weist die Führung einen Innendurchmesser zwischen 3 Millimeter und 9 Millimeter, insbesondere von 7 Millimetern, auf. Durch eine derartige Wahl des Innendurchmessers kann erreicht werden, dass nur eine geringe Kraft benötigt wird, um den Pfannenteil mit dem Verankerungsteil fest zu verbinden, wobei die erforderliche Kraft insbesondere zwischen 15 kp und 22 kp (147,10 Newton bis 215,75 Newton) beträgt, während Mindestkraft zum Lösen der festen Verbindung zwischen Pfannenteil und Verankerungsteil mindestens 20 kp (196,13 Newton) beträgt und die Vorgaben somit erfüllt.

Gemäss einem weiteren bevorzugten Ausführungsbeispiel der Erfindung weist die Führung auf der von der Einfuhrseite abgewandten Seite eine Ausgangsöffnung auf. Durch diese Ausgangsöffnung können vorteilhafterweise in der Führung befindliche Substanzen, wie beispielsweise Gewebeteile oder Blut, abfliessen, so dass eine ungehinderte Einfuhr und ein ungehindertes Halten des Stifts in der Führung und ein ungehindertes festes Verbinden des Pfannenteils mit dem Verankerungsteil erfolgen kann.

Gemäss einem weiteren Ausführungsbeispiel der Erfindung weist das Verankerungsteil eine Auflagefläche auf, die mit einer Öffnung zur Einfuhr des Stiftes in die Führung versehen ist. Die Öffnung zur Einfuhr des Stiftes bildet vorzugsweise die Einfuhrseite der Führung. Die Auflagefläche ist bevorzugterweise derart ausgebildet, dass das Pfannenteil flächig auf der Auflagefläche anbringbar ist. Die Krümmungsradien von Pfannenteil und Auflagefläche sind an der Verbindungs- bzw. Kopplungsstelle vorzugsweise gleich, wodurch eine gute Auflage erzielt werden kann. Die Auflagefläche bietet einen weiteren Halt für den Pfannenteil und schützt die Führung vor dem Eindringen von Substanzen wie Gewebeteilen und Blut. Durch die flächige Anbringung wird ein Verschieben bzw. "Wackeln" des Pfannenteils auf der Auflagefläche weitestgehend verhindert.

Gemäss einem bevorzugten Ausführungsbeispiel der Erfindung beträgt der Abstand von der Auflagefläche zu der Verbindungseinrichtung zwischen 3 Millimeter und 12 Millimeter, insbesondere 4 Millimeter. Der Abstand entspricht im Wesentlichen dem oben genannten bevorzugten Abstand zwischen dem Pfannenteil und dem ersten Teil der Verbindungseinrichtung. Durch den gewählten Abstand kann eine sichere Führung und ein sicheres Halten des Stiftes in der Führung sichergestellt werden. Ferner können die Kräfte, die zum festen Verbinden des Pfannenteils mit dem Verankerungsteil aufgebracht werden müssen, minimiert werden, während die notwendige Kraft zum Lösen der festen Verbindung eingehalten oder überschritten werden kann.

Das Verfahren zum Zusammenbau einer Prothese der oben genannten Art zeichnet sich dadurch aus, dass wenigstens ein Stift in die wenigstens eine Führung des Verankerungsteils eingeführt wird, bis eine feste Verbindung zwischen dem Pfannenteil und dem Verankerungsteil durch die Verbindungseinrichtung gebildet wird. Hierdurch kann verhindert werden, dass bei einem Öffnen der Verbindungseinrichtung das Pfannenteil nicht mehr in Wirkverbindung mit dem Verankerungsteil steht, denn der dem Pfannenteil zugeordnete Stift wird weiterhin durch die Führung des Verankerungsteils gehalten.

Gemäss einer bevorzugten Ausführungsform Verfahrens schnappt zum Bilden der festen Verbindung zwischen dem Pfannenteil und dem Verankerungsteil ein erstes, an dem Stift angeordnetes Teil in ein zweites, an der Führung angeordnetes Teil der Verbindungseinrichtung ein. Auf diese Weise kann eine besonders feste Verbindung zwischen dem Pfannenteil und dem Verankerungsteil erzielt werden.

### Kurze Beschreibung der Zeichnungen

Weitere vorteilhafte Ausgestaltungen der Erfindung ergeben sich aus den Unteransprüchen und aus den anhand der Zeichnung nachfolgend dargestellten Ausführungsbeispielen. Es zeigen:
Figur 1 eine Schnittdarstellung eines bevorzugten Ausführungsbeispiels einer erfindungsgemässen Prothese,
Figur 2 eine vergrösserte Darstellung eines Teils des in der Figur 1 dargestellten bevorzugten Ausführungsbeispiels der erfindungsgemässen Prothese und
Figur 3 eine Schnittdarstellung einer bevorzugten Ausführungsform einer erfindungsgemässen Prothese mit Befestigungsmitteln.

In den Figuren bezeichnen gleiche Bezugszeichen strukturell bzw. funktionell gleichwirkende Komponenten.

### Weg(e) zur Ausführung der Erfindung

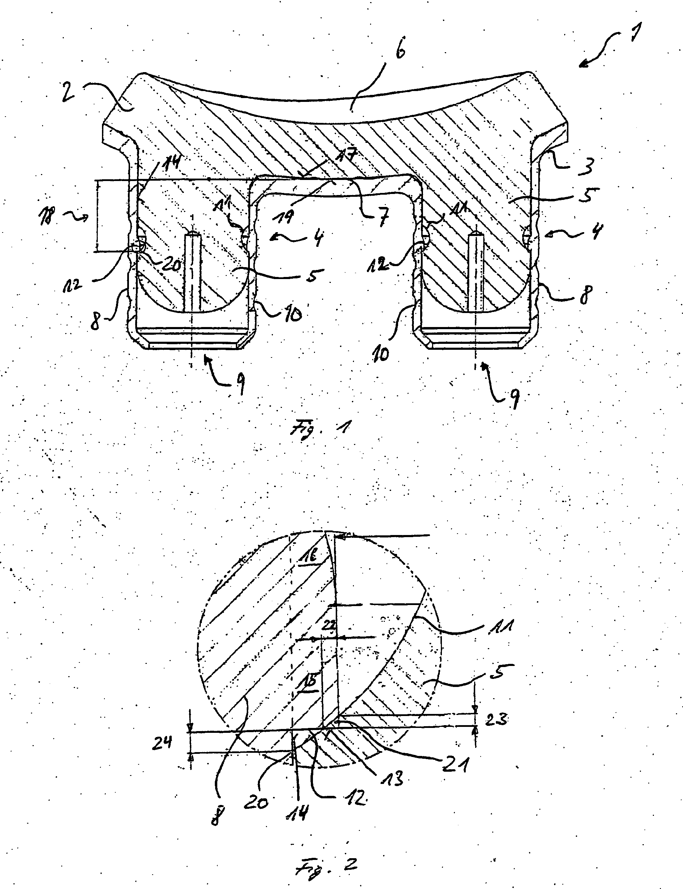

Figur 1 zeigt eine Schnittdarstellung eines bevorzugten Ausführungsbeispiels einer erfindungsgemässen Prothese 1. Die Prothese 1 kann insbesondere in den Teil eines Schulterblatts eingepflanzt werden, der der Aufnahme des Kopfes eines Oberarmknochens dient. Es handelt sich somit insbesondere um eine Glenoid-Prothese. Selbstverständlich kann die Prothese 1 auch in ein Hüftgelenk zur Aufnahme des Kopfes eines Oberschenkelknochens eingesetzt werden.

Die Prothese 1 umfasst ein Pfannenteil 2 zur Aufnahme eines Gelenkkopfes und ein Verankerungsteil 3 zum Verankern der Prothese 1 in einem Knochen. Es ist eine Verbindungseinrichtung 4 zum festen Verbinden des Pfannenteils 2 mit dem Verankerungsteil 3 vorgesehen. Dem Pfannenteil 2 sind zwei Stifte 5 zugeordnet, wobei das Pfannenteil 2 und die Stifte 5 vorzugsweise einstückig ausgeführt sind. Auf diese Weise kann ein Eindringen von Schmutz zwischen den Stiften 5 und dem Pfannenteil 2 verhindert werden. Das Pfannenteil 2 und/oder die Stifte 5 sind vorzugsweise aus einem Kunststoff, insbesondere aus Polyethylen, gebildet. Das Verankerungsteil 3 ist bevorzugterweise aus einem Metall gebildet.

Es kann auch nur ein Stift 5 oder es können mehr als zwei Stifte 5, insbesondere drei Stifte 5, vorgesehen sein. Das Versehen mit mehr als einem Stift 5 hat den Vorteil, dass die Rotationsstabilität der Verbindung von Pfannenteil 2 und Verankerungsteil 3 hoch ist. Die Stifte 5 sind bevorzugt symmetrisch um das Zentrum des Pfannenteils 2 herum angeordnet. Das Pfannenteil 2 weist eine Mulde 6 zur Aufnahme eines Gelenkkopfes auf und die Stifte 5 sind auf der der Mulde 6 gegenüberliegenden Seite 7 des Pfannenteils 2 angeordnet.

Das Verankerungsteil 3 weist zwei Führungen 8 auf, die derart ausgestaltet sind, dass die Stifte 5 im Wesentlichen kippsicher in den Führungen 8 gehalten werden können. Sind mehr oder weniger als zwei Stifte 5 vorgesehen, so entspricht die Anzahl der Führungen 8 der Anzahl der Stifte 5. Die Stifte 5 und die Führung 8 sind parallel zueinander ausgerichtet und im Wesentlichen zylindrisch ausgebildet, wobei der Innendurchmesser der Führungen 8 im Wesentlichen dem Aussendurchmesser der Stifte 5 entspricht, so dass ein sicherer Halt der Stifte 5 in den Führungen 8 gegeben ist. Ferner kann vorteilhafterweise ein Eindringen von Schmutz bzw. Substanzen wie beispielsweise Blut und Gewebeteilchen in die Prothese 1 verhindert werden. Hierdurch wird wiederum eine sichere feste Verbindung des Pfannenteils 2 mit dem Verankerungsteil 3 gewährleistet.

Die Führungen 8 weisen an der der nicht näher bezeichneten Einfuhrseite für die Stifte 5 gegenüberliegenden Seite Ausgangsöffnungen 9 auf, durch die Schmutz und Substanzen wie Blut und Gewebeteile ablaufen bzw. abgeführt werden können.

Zur besseren Verankerung der Führungen 8 in einem Knochen können an dessen Aussenseite umlaufende Nuten 10 oder dergleichen vorgesehen sein.

Die Verbindungseinrichtung 4, die vorzugsweise in Bezug auf jeden Stift 5 bzw. jede Führung 8 vorgesehen ist, umfasst einen ersten Teil 11, der als Vertiefung, vorzugsweise als ringförmige Nut, in den Stiften 5 ausgeführt ist und einen zweiten Teil 12, der als Verdickung, vorzugsweise ringförmig, in den Führungen 8 vorgesehen ist.

Figur 2 zeigt einen vergrösserten Ausschnitt der in der Figur 1 dargestellten Prothese, der die Verbindung zwischen dem ersten Teil 11 und dem zweiten Teil 12 der Verbindungseinrichtung 4 zeigt. Der zweite Teil 12 ragt radial in die Führung 8 hinein, wobei die der Ausgangsöffnung 9 der Führung 8 zugewandte Kante 13 des zweiten Teils 12 bevorzugterweise in einem im Wesentlichen rechten oder zumindest steilen Winkel von der Innenseite 14 der Führung absteht. In vom Betrachter aus gesehen vertikaler bzw. axialer Richtung verläuft der zweite Teil 12 bevorzugt von der Kante 13 aus in Richtung zum Pfannenteil 2 in einem ersten Bereich 15 parallel zur Innenseite der Führung 8 und verjüngt sich in einem zweiten Bereich 16, der sich in vertikaler bzw. axialer Richtung an den ersten Bereich 15 anschliesst, bis er in die Innenseite 14 der Führung 8 übergeht.

Das Verankerungsmittel 3 weist eine Auflagefläche 17 auf, die vorzugsweise derart ausgebildet ist, dass das Pfannenteil 3 flächig auf der Auflagefläche 17 angebracht werden kann. Hierdurch kann vorteilhafterweise ein genauer Sitz des Pfannenteils 2 auf dem Verankerungsteil 3 erzielt werden. Die Krümmungsradien von Pfannenteil 3 und Auflagefläche 17 sind an der Verbindungs- bzw. Kopplungsstelle vorzugsweise gleich, wodurch eine gute Auflage erzielt werden kann.

Der erste Teil 11 der Verbindungseinrichtung 4 ist in einem Abstand 18 zum Pfannenteil 2 angeordnet, wobei der Abstand 18 zwischen der Mitte der unteren Seite 19 des Pfannenteils 2 zu dem der Ausgangsöffnung 9 zugewandten Ende 20 des vorzugsweise als Nut ausgeführten ersten Teils 11 vorzugsweise zwischen 3 Millimeter und 12 Millimeter, bevorzugterweise 4 Millimeter beträgt. Der nicht näher bezeichnete Abstand von der Auflagefläche 17 zu der Kante 13 des zweiten Teils 12 entspricht im Wesentlichen dem Abstand 18.

Da die Führung 8 und somit auch der zweite Teil 12 der Verbindungseinrichtung 4 aus Metall bestehen und der Stift 5 aus einem Kunststoff, insbesondere Polyethylen, gebildet ist, kommt es bei der Verbindung des ersten Teils 11 mit dem zweiten Teil 12 bzw. bei der Verbindung des Stifts 5 mit der Führung 8 zu einer Verformung des Stifts 5, die dadurch bedingt ist, dass die von der Innenseite 14 der Führung 8 beabstandete und der Ausgangsöffnung 9 zugewandte Kante 21 des zweiten Teils 12 sich in den Stift 5 presst und diesen somit verformt. Die radiale Einpresstiefe 22 beträgt für das in den Figuren 1 und 2 dargestellte Ausführungsbeispiel ungefähr 0,05 Millimeter. Die vertikale bzw. axiale Einpresstiefe 23 beträgt für das in den Figuren 1 und 2 dargestellte Ausführungsbeispiel ungefähr 0,04 Millimeter. Das Eindringen der Kante 21 des zweiten Teils 12 in den Stift 5 verhindert, dass der zweite Teil 12 bis an das der Ausgangsöffnung 9 zugewandte Ende des ersten Teils 11 heranreicht. Dies hat zur Folge, dass der Abstand der Auflagefläche 17 zum Rand 13 des zweiten Teils 12 bei dem gezeigten Ausführungsbeispiel um eine Differenz 24 von ungefähr 0,07 Millimeter kleiner ist als der Abstand 18 von der unteren Seite 19 des Pfannenteils 2 zum unteren Rand 20 des ersten Teils 11.

Die Führung 8 weist an der Stelle des ersten Bereichs 15 des als Verdickung ausgeführten zweiten Teils 12 der Verriegelungseinrichtung 4 bei dem in den Figuren 1 und 2 dargestellten Ausführungsbeispiel bevorzugterweise einen Innendurchmesser von 6,82 Millimetern auf, wobei vorzugsweise der Innendurchmesser der Führung 8 ohne Berücksichtigung der Verdickung 7 Millimeter und die Breite der Verdickung 0,18 Millimeter betragen. Der Stift 5 weist an der Stelle des als Öffnung ausgeführten ersten Teils 11 vorzugsweise einen Aussendurchmesser von 6,70 Millimetern auf, wobei bevorzugterweise der Aussendurchmesser des Stifts 5 ohne Berücksichtigung der Öffnung 7 Millimeter und die Breite der Öffnung 0,3 Millimeter betragen. Bevorzugt beträgt die Breite der Verdickung 12 ungefähr 5 Prozent des Innendurchmessers der Führung 8 ohne Berücksichtigung der Verdickung 12 und die Breite der Öffnung 11 ungefähr 5 Prozent des Aussendurchmessers des Stifts 5 ohne Berücksichtigung der Öffnung 11. Die Verbindungseinrichtung 4 hat vorzugsweise einen Durchmesser von 3 bis 9 Millimetern, insbesondere von 6 bis 7 Millimetern.

Die in Bezug auf das in den Figuren 1 und 2 dargestellte Ausführungsbeispiel angegebenen Abmessungen ermöglichen eine feste Verbindung zwischen dem Pfannenteil 2 und dem Verankerungsteil 3 durch das Aufbringen geringer Einpresskräfte, während gleichzeitig die notwendigen Auspresskräfte zum Lösen der festen Verbindung eingehalten werden.

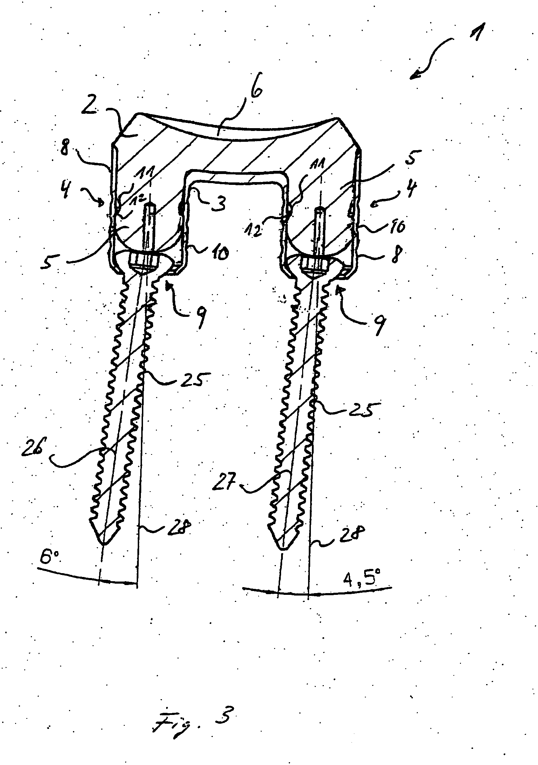

Figur 3 zeigt eine Schnittdarstellung einer Prothese 1, wie sie in der Figur 1 dargestellt ist, bei der zusätzlich durch die Ausgangsöffnungen 9 hindurchgreifende Befestigungsmittel 25, insbesondere Schrauben, zur Befestigung des Verankerungsteils 3 in einem Knochen vorgesehen sind. Die Achsen 26, 27 der Befestigungsmittel verlaufen vorzugsweise nicht parallel zu den Achsen 28 der Stifte 5. Auf diese Weise kann eine verbesserte Verankerung erzielt werden. Die Neigung der Achsen 26, 27 der Befestigungsmittel 25 gegenüber den Achsen 28 der Stifte 5 beträgt vorzugsweise zwischen 4,5 und 6 Grad. Ein besonders guter Halt im Knochen kann erzielt werden, wenn die Neigung der Achsen 26, 27 der Befestigungsmittel 25 gegenüber den Achsen 28 der Stifte 5 unterschiedlich gross ist. So kann beispielsweise die Neigung der Achse 26 eines ersten Befestigungsmittels 25 gegenüber der Achse 28 eines ersten Stiftes 5 6 Grad und die Neigung der Achse 27 eines zweiten Befestigungsmittels 25 gegenüber der Achse 28 eines zweiten Stiftes 5 4,5 Grad betragen.

Zur Implantation bzw. zum Einpflanzen der Prothese 1 in einen Knochen werden in einem ersten Schritt die Führungen 8 in den Knochen eingebracht. Dies kann beispielsweise mittels Einschrauben der Schrauben 25 und/oder durch das Aufbringen einer Kraft beispielsweise mittels eines Hammers erfolgen. In einem weiteren Schritt wird das Pfannenteil 2 mit dem Verankerungsteil 3 verbunden, indem die Stifte 5 in die Führungen 8 eingeführt werden. Die Stifte 5 werden in die Führungen 8 solange eingeführt, bis durch die Verbindungseinrichtung 4 eine feste Verbindung zwischen dem Pfannenteil 2 bzw. den Stiften 5 und dem Verankerungsteil 3 bzw. den Führungen 8 gebildet worden ist. Die Stifte 5 werden beim Einführen in die Führungen 8 über die jeweiligen Verdickungen 12 der Verbindungseinrichtungen 4 hinübergeführt. Dabei rasten bzw. schnappen die Verdickungen 12 in die Öffnungen 11 ein und schaffen auf diese Weise eine feste, d.h. nicht leicht lösbare, Verbindung zwischen den Stiften 5 und den Führungen 8 und somit zwischen dem Pfannenteil 2 und dem Verankerungsteil 3. Selbstverständlich kann ein derartiger Zusammenbau von Pfannenteil 2 und Verankerungsteil 3 auch ausserhalb eines menschlichen oder tierischen Körpers erfolgen.

Die Verdickung 12, die in die Öffnung 11 eingerastet bzw. eingeschnappt ist, verhindert, dass die Stifte 5 einfach aus den Führungen 8 wieder hinausgleiten können. Wird dennoch, beispielsweise durch eine Schaukelbewegung, die Verdickung 12 von der Öffnung 11 getrennt, so verbleiben die Stifte 5 vorteilhafterweise in den Führungen 8 und werden von diesen gehalten, d.h. die Verbindung von Pfannenteil 2 und Verankerungsteil 3 wird nicht vollständig gelöst und bleibt reparabel. Eine erneute feste Verbindung kann einfach erzielt werden, indem das Pfannenteil 2 und somit die Stifte 5 soweit in Richtung der Ausgangsöffnungen 9 gedrückt werden, dass die Verdickungen 12 wieder in die Öffnungen 11 einrasten.

## Patentansprüche

1. Prothese, insbesondere Schulterprothese, mit einem Pfannenteil (2) zur Aufnahme eines Gelenkkopfs, einem Verankerungssteil (3) zum Verankern der Prothese (1) in einem Knochen und einer Verbindungseinrichtung (4) zum festen Verbinden des Pfannenteils (2) mit dem Verankerungsteil (3), wobei
A) dem Pfannenteil (2) mehrere, insbesondere zwei, Stifte (5) zugeordnet sind und jeder Stift (5) eine Längsachse aufweist, und dass das Verankerungsteil (3) mehrere, insbesondere zwei, Führungen (8) zur kippsicheren Führung der Stifte (5) aufweist; wobei
B) die Führungen (8) Ausgangsöffnungen (9) aufweisen und die Führungen (8) derart ausgestaltet sind, dass die Stifte (5) im Wesentlichen kippsicher in den Führungen (8) haltbar sind, **dadurch gekennzeichnet, dass**
C) in den Ausgangsöffnungen (9) der mehreren Führungen (8) ein Befestigungsmittel (25), insbesondere eine Schraube, mit einer Längsachse zur Befestigung des Verankerungsteils (3) in einem Knochen vorgesehen ist; und
D) die Längsachse (26;27) des jeweiligen Befestigungsmittel (25) gegenüber den Längsachsen (28) der mehreren Stifte (5) neigbar sind.

2. Prothese nach Anspruch 1, **dadurch gekennzeichnet, dass** das Pfannenteil (2) und die Stifte (5) einstückig ausgeführt sind.

3. Prothese nach Anspruch 1 oder 2, **dadurch gekennzeichnet, dass** die Führung (8) und der Stift (5) im Wesentlichen zylindrisch ausgebildet sind, wobei der Innendurchmesser der Führung (8) im Wesentlichen dem Aussendurchmesser des Stifts (5) entspricht.

4. Prothese nach einem der vorhergehenden Ansprüche, **dadurch gekennzeichnet, dass** das Pfannenteil (2) eine Mulde (6) zur Aufnahme eines Gelenkkopfs aufweist und der Stift (5) auf der der Mulde (6) gegenüberliegenden Seite (17) des Pfannenteils (2) angeordnet ist.

5. Prothese nach einem der vorhergehenden Ansprüche, **dadurch gekennzeichnet, dass** die Verbindungseinrichtung (4) als Einschnappeinrichtung ausgeführt ist.

6. Prothese nach einem der vorhergehenden Ansprüche, **dadurch gekennzeichnet, dass** ein erster Teil (11) der Verbindungseinrichtung (4) an dem Stift (5) in einem Abstand (18) zu dem Pfannenteil (2) angeordnet ist und dass ein zweiter Teil (12) der Verbindungseinrichtung (4) in der Führung (8) in einem Abstand zu einer Einfuhrseite der Führung (8) angeordnet ist.

7. Prothese nach Anspruch 6, **dadurch gekennzeichnet, dass** der Abstand (18) von dem Pfannenteil (2) zu der Verbindungseinrichtung (4) zwischen 3 mm und 12 mm, insbesondere von 4 mm, beträgt.

8. Prothese nach Anspruch 6 oder 7, **dadurch gekennzeichnet, dass** der erste Teil (11) der Verbindungseinrichtung (4) als Öffnung, insbesondere als im Wesentlichen ringförmige Nut, ausgebildet ist.

9. Prothese nach Anspruch 8, **dadurch gekennzeichnet, dass** die Ränder der Öffnung (4) eine Fase aufweisen.

10. Prothese nach einem der Ansprüche 6 bis 9, **dadurch gekennzeichnet, dass** der zweite Teil (12) der Verbindungseinrichtung (4) als Verdickung, insbesondere als im Wesentlichen ringförmiger Wulst, ausgebildet ist.

11. Prothese nach Anspruch 10, **dadurch gekennzeichnet, dass** die Führung (8) einen Innendurchmesser zwischen 3 mm und 9 mm, insbesondere von 7 mm, aufweist.

12. Prothese nach einem der vorhergehenden Ansprüche, **dadurch gekennzeichnet, dass** die Führung (8) auf der von der Einfuhrseite abgewandeten Seite die Ausgangsöffnung (9) aufweist.

13. Prothese nach einem der vorhergehenden Ansprüche, **dadurch gekennzeichnet, dass** das Verankerungsteil (3) eine Auflagefläche (19) aufweist, die mit einer Öffnung zur Einfuhr des Stiftes in die Führung versehen ist.

14. Prothese nach Anspruch 13, **dadurch gekennzeichnet, dass** die Auflagefläche (19) derart ausgebildet ist, dass das Pfannenteil (2) flächig auf der Auflagefläche (19) anbringbar ist.

15. Prothese nach Anspruch 13 oder 14, **dadurch gekennzeichnet, dass** der Abstand von der Auflagefläche (19) zu der Verbindungseinrichtung (4) zwischen 3 mm und 12 mm, insbesondere 4 mm beträgt.

16. Prothese nach einem der vorhergehenden Ansprüche, **dadurch gekennzeichnet, dass** das Pfannenteil (2) und/oder der Stift (5) aus einem Kunststoff, insbesondere aus Polyethylen, ausgebildet sind.

17. Prothese nach einem der vorhergehenden Ansprüche, **dadurch gekennzeichnet, dass** das Verankerungsteil (3) aus Metall ausgebildet ist.

18. Prothese nach einem der vorhergehenden Ansprüche, **dadurch gekennzeichnet, dass** die Stifte (5) und die Führungen (8) parallel ausgerichtet sind.

## Claims

1. Prosthesis, in particular shoulder prosthesis, with a socket part (2) to receive a joint head, an anchoring part (3) to anchor the prosthesis (1) in a bone and a connecting device (4) to securely connect the socket part (2) with the anchoring part (3), wherein
A) the socket part (2) is assigned several, in particular two pins (5) and each pin (5) has a longitudinal axis, and that the anchoring part (3) has several, in particular two guides (8) for a tilt-proof guiding of the pins (5), wherein
B) the guides (8) are provided with outlet openings (9) and the guides (8) are configured so that the pins (5) can be held in the guides (8) in an essentially tilt-proof manner,
**characterized in that**
C) a fastening means (25) especially a screw with a longitudinal axis, is provided in the outlet openings (9) of the several guides (8) to fasten the anchoring part (3) in a bone; and
D) the longitudinal axis (26;27) of the respective fastening means (25) is inclinable relative to the longitudinal axes (28) of the several pins (5).

2. Prosthesis according to claim 1, **characterized in that** the socket part (2) and the pins (5) are implemented in a single piece.

3. Prosthesis according to claim 1 or 2, **characterized in that** the guide (8) and the pin (5) are conformed in an essentially cylindrical fashion, where the inner diameter of the guide (8) essentially corresponds to the outer diameter of the pin (5).

4. Prosthesis according to one of the previous claims, **characterized in that** the socket part (2) has a pan (6) to receive a joint head and the pin (5) is arranged on the side (17) of the socket part (2) opposite the pan (6).

5. Prosthesis according to one of the previous claims, **characterized in that** the connecting device (4) is executed as a snap-in device.

6. Prosthesis according to one of the previous claims, **characterized in that** a first portion (11) of the connecting device (4) is arranged on the pin (5) at a distance (18) to the socket part (2), and that a second portion (12) of the connecting device (4) is arranged in the guide (8) at a distance to the inlet side of the guide (8).

7. Prosthesis according to claim 6, **characterized in that** the distance (18) from the socket part (2) to the connecting device (4) amounts to between 3 mm and 12 mm, in particular to 4 mm.

8. Prosthesis according to claim 6 or 7, **characterized in that** the first portion (11) of the connecting device (4) is conformed as an opening, in particular as an essentially annular groove.

9. Prosthesis according to claim 8, **characterized in that** the rims of the opening (4) present a chamfer.

10. Prosthesis according to one of the claims from 6 to 9, **characterized in that** the second portion (12) of the connecting device (4) is conformed as a thickening, in particular as an essentially ring-shaped bulge.

11. Prosthesis according to claim 10, **characterized in that** the guide (8) has an inner diameter of between 3 mm and 9 mm, in particular of 7 mm.

12. Prosthesis according to one of the previous claims, **characterized in that** on the side turned away from the inlet side, the guide (8) has the outlet opening (9).

13. Prosthesis according to one of the previous claims, **characterized in that** the anchoring part (3) has a bearing surface (19) fitted with an opening to insert the pin into the guide.

14. Prosthesis according to claim 13, **characterized in that** the bearing surface (19) is conformed so that the socket part (2) can be applied to the bearing surface (19) in a planar manner.

15. Prosthesis according to claim 13 or 14, **characterized in that** the distance from the bearing surface (19) to the connecting device (4) amounts to between 3 mm and 12 mm, in particular to 4 mm.

16. Prosthesis according to one of the previous claims, **characterized in that** the socket part (2) and/or the pin (5) are made of a plastic material, in particular of polyethylene.

17. Prosthesis according to one of the previous claims, **characterized in that** the anchoring part (3) is made of metal.

18. Prosthesis according to one of the previous claims, **characterized in that** the pins (5) and the guides (8) are oriented in a parallel manner.

## Revendications

1. Prothèse, en particulier prothèse d'épaule, comprenant un élément glénoïdal (2) permettant de loger une tête d'articulation, un élément d'ancrage (3) permettant d'ancrer la prothèse (1) dans un os et un dispositif de liaison (4) prévu pour lier solidement l'élément glénoïdal (2) avec l'élément d'ancrage (3), dans laquelle
A) plusieurs broches (5), en particulier deux, sont associées à l'élément glénoïdal (2) et chaque broche (5) présente un axe longitudinal, et l'élément d'ancrage (3) présente plusieurs, en particulier deux, guidages (8) pour le guidage stable au renversement des broches (5) ; dans laquelle
B) les guidages (8) présentent des ouvertures de sortie (9) et les guidages (8) sont conçus de telle sorte que les broches (5) peuvent être maintenues dans les guidages (8) de manière essentiellement stable au renversement, **caractérisée en ce que**
C) un moyen de fixation (25), en particulier une vis présentant un axe longitudinal pour la fixation de l'élément d'ancrage (3) dans un os est prévu dans les ouvertures de sortie (9) d'une pluralité de guidages (8) ; et
D) l'axe longitudinal (26 ; 27) du moyen de fixation respectif (25) peut être incliné par rapport aux axes longitudinaux (28) d'une pluralité de broches (5).

2. Prothèse selon la revendication 1, **caractérisée en ce que** l'élément glénoïdal (2) et les broches (5) sont formés d'une seule pièce.

3. Prothèse selon la revendication 1 ou 2, **caractérisée en ce que** le guidage (8) et la broche (5) sont réalisés essentiellement de forme cylindrique, dans laquelle le diamètre intérieur du guidage (8) correspond essentiellement au diamètre extérieur de la broche (5).

4. Prothèse selon l'une quelconque des revendications précédentes, **caractérisée en ce que** l'élément glénoïdal (2) présente une cavité (6) pour loger une tête d'articulation et la broche (5) est disposée sur le côté opposé (17) à la cavité (6) de l'élément glénoïdal (2).

5. Prothèse selon l'une quelconque des revendications précédentes, **caractérisée en ce que** le dispositif de liaison (4) est réalisé sous la forme d'un dispositif à encliquetage.

6. Prothèse selon l'une quelconque des revendications précédentes, **caractérisée en ce qu'**une première partie (11) du dispositif de liaison (4) est disposée sur la broche (5) à une certaine distance (18) par rapport à l'élément glénoïdal (2) et **en ce qu'**une deuxième partie (12) du dispositif de liaison (4) est disposée dans le guidage (8) à une certaine distance par rapport à un côté d'introduction du guidage (8).

7. Prothèse selon la revendication 6, **caractérisée en ce que** la distance (18) de l'élément glénoïdal (2) au dispositif de liaison (4) est comprise entre 3 mm et 12 mm, distance qui mesure en particulier 4 mm.

8. Prothèse selon la revendication 6 ou 7, **caractérisée en ce que** le première partie (11) du dispositif de liaison (4) est réalisée sous la forme d'un évidement, en particulier sous la forme d'une rainure essentiellement annulaire.

9. Prothèse selon la revendication 8, **caractérisée en ce que** les bords de l'évidement (4) présentent un chanfrein.

10. Prothèse selon l'une quelconque des revendications 6 à 9, **caractérisée en ce que** la deuxième partie (12) du dispositif de liaison (4) est réalisée sous la forme d'un bourrelet, en particulier essentiellement sous la forme d'un collet annulaire.

11. Prothèse selon la revendication 10, **caractérisée en ce que** le guidage (8) présente un diamètre intérieur compris entre 3 mm et 9 mm, diamètre qui mesure en particulier 7 mm.

12. Prothèse selon l'une quelconque des revendications précédentes, **caractérisée en ce que**, sur le côté opposé au côté d'introduction, le guidage (8) présente l'ouverture de sortie (9).

13. Prothèse selon l'une quelconque des revendications précédentes, **caractérisée en ce que** l'élément d'ancrage (3) présente une surface d'appui (19), qui est pourvue d'un évidement pour l'introduction de la broche dans le guidage.

14. Prothèse selon la revendication 13, **caractérisée en ce que** la surface d'appui (19) est réalisée de telle sorte que l'élément glénoïdal (2) peut être placé sur la surface d'appui (19) avec un contact surfacique.

15. Prothèse selon la revendication 13 ou 14, **caractérisée en ce que** la distance de la surface d'appui (19) au dispositif de liaison (4) est comprise entre 3 mm et 12 mm, distance qui mesure en particulier 4 mm.

16. Prothèse selon l'une quelconque des revendications précédentes, **caractérisée en ce que** l'élément glénoïdal (2) et/ou la broche (5) est fait d'une matière plastique, en particulier de polyéthylène.

17. Prothèse selon l'une quelconque des revendications précédentes, **caractérisée en ce que** l'élément d'ancrage (3) est fait de métal.

18. Prothèse selon l'une quelconque des revendications précédentes, **caractérisée en ce que** les broches (5) et les guidages (8) sont orientés parallèlement.
